# FASCICULE DE BREVET EUROPEEN

(11) **EP 2 804 658 B1**
(45) Date de publication et mention de la délivrance du brevet: **30.10.2019**
(21) Numéro de dépôt: 13700314.1
(22) Date de dépôt: 16.01.2013
(51) Int. Cl.: A61M 31/00, A61F 13/20, A61F 13/26, A61K 9/00, A61K 9/70

(54) **DISPOSITIF SANITAIRE POUR L'APPLICATION D'UN PRINCIPE ACTIF AU SEIN D'UNE CAVITÉ NATURELLE**
SANITÄRVORRICHTUNG ZUM ANWENDEN EINES WIRKUNGSPRINZIPS IN EINER KÖRPERHÖHLE
SANITARY DEVICE FOR APPLYING AN ACTIVE PRINCIPLE WITHIN A BODY CAVITY

(30) Priorité: 19.01.2012 FR 1250565
(43) Date de publication de la demande: 26.11.2014
(73) Titulaire: NUTRINOV, 35530 Noyal-sur-Vilaine (FR)
(72) Inventeur: EFSTATHIOU, Théo, 35740 Pace (FR); MORIN, Christophe, 53300 La Haie Traversaire (FR)
(74) Mandataire: Vidon Brevets & Stratégie
(86) Numéro de dépôt international: PCT/EP2013/050746
(87) Numéro de publication internationale: WO 2013/107770

(56) Documents cités:
- US-A- 4 398 532
- US-A- 5 292 307
- US-A1- 2003 139 709
- US-A1- 2006 216 334

## Description

### 1. Domaine de l'invention

Le domaine de l'invention est celui des dispositifs sanitaires utilisés pour appliquer un principe actif dans une cavité naturelle du corps humain ou animal.

### 2. Art antérieur

Le traitement de certaines affections affectant des cavités naturelles du corps humain, ou la prévention de telles affections peut consister à administrer au patient un principe actif par voie générale (forme orale ou injectable). Toutefois, cette option présente quelques inconvénients. Notamment, le patient ingère une quantité de principe actif qui peut être plus importante que cela est nécessaire. Cela engendre une exposition superflue de l'organisme entier à ces principes actifs ce qui peut être particulièrement gênant lorsqu'il s'agit de substances chimiques. Or, prendre des principes actifs, qu'ils soient thérapeutiques ou prophylactiques, tels que notamment des médicaments n'est pas un geste anodin : outre l'effet que la prise apporte, les effets négatifs sur l'ensemble des organes est également à prendre en compte.

Il est, par exemple, notoire que le foie, organe de détoxification des composés exogènes, est lentement détruit par la prise de médicaments sur le long terme. Ainsi, les patients prenant un traitement sur le long terme développent fréquemment des pathologies hépatiques, telles que la cirrhose médicamenteuse. Les reins et le système digestif peuvent également souffrir de manière importante des traitements chroniques. Enfin, lorsqu'il s'agit d'hormones, une très faible quantité suffit à impacter l'organisme entier du patient (foie, cerveau, cellules graisseuses, organes génitaux et reproducteurs etc...).

Une solution pour pallier ces inconvénients consiste à délivrer de manière locale des principes actifs utilisés à titre préventif ou thérapeutique en les incluant dans des compositions se présentant sous forme de pastilles, de gels, de films, de suppositoires, de mousses...

En gynécologie, on connaît notamment l'utilisation de film pour appliquer à la surface des muqueuses du vagin des principes actifs telles que des hormones, des antibiotiques, des spermicides, des antifongiques etc...

Le document US 4,398,532 décrit un dispositif d'insertion d'un diaphragme et d'un spermicide dans le vagin. Le piston du dispositif sort de façon minimale hors du tube en mode d'insertion afin d'éviter toute blessure de la paroi vaginale.

Les documents US 2006/0216334 et US 5,292,307 décrivent deux types de dispositif d'insertion d'un médicament dans les cavités naturelles d'un patient.

Toutefois, de tels films doivent être conformés en tubes (par exemple par enroulement) avant d'être introduits avec le doigt dans le vagin. Il en résulte que le traitement est boudé ou rapidement arrêté par la patiente dès les premiers signes d'amélioration. Or, l'observance d'un traitement est un paramètre important dans le succès de celui-ci. De plus, une mauvaise observance conduit systématiquement à une augmentation de la résistance aux traitements chez les bactéries, les virus et les champignons.

Il existe donc un besoin pour un moyen dédié à l'application simple et efficace d'un principe actif à l'intérieur d'une cavité naturelle du corps humain, notamment le vagin, permettant d'améliorer le confort d'application et donc l'observance du traitement proposé par un tel principe actif.

### 3. Objectifs de l'invention

L'invention a donc pour objectif de fournir un dispositif permettant d'appliquer de manière locale un principe actif dans une cavité naturelle du corps humain ou animal.

L'invention a encore pour objectif de permettre, dans au moins un mode de réalisation, de proposer un tel dispositif qui soit d'utilisation simple.

Un autre objectif de l'invention est de concevoir, dans au moins un mode de réalisation, un tel dispositif dont la fabrication est simple et économique.

### 4. Exposé de l'invention

Ces objectifs, ainsi que d'autres qui apparaîtront par la suite, sont atteints à l'aide d'un dispositif sanitaire d'application d'au moins un principe actif à l'intérieur d'une cavité naturelle humaine ou animale comme définit dans la revendication indépendante 1 et dans les revendications dépendantes 2 à 14.

Selon l'invention, un tel dispositif comprend au moins un film biodégradable contenant ledit au moins un principe actif et un applicateur incluant :
- un corps principal tubulaire présentant une première ouverture à son extrémité proximale et une seconde ouverture à son extrémité distale; et
- un corps éjecteur comprenant une portion distale dont au moins une partie de la surface forme un support pour l'au moins un film biodégradable et dont l'extrémité est arrondie,
ledit corps éjecteur étant mobile de manière coulissante à l'intérieur dudit corps principal entre au moins une position dans laquelle ladite portion distale dudit corps éjecteur se trouve à l'intérieur dudit corps principal et une position d'application dans laquelle ladite portion distale se trouve à l'extérieur dans le prolongement axial dudit corps principal.

Ainsi, l'invention repose sur une approche tout à fait originale proposant l'utilisation d'un applicateur intégrant un corps éjecteur formant piston pour appliquer dans une cavité naturelle un film biodégradable renfermant au moins un principe actif.

Par « biodégradable », on comprend que le film peut être dégradé naturellement une fois qu'il est appliqué dans une cavité naturelle du corps humain dans les conditions de température et d'humidité régnant à l'intérieur de ladite cavité.

De par la simplicité d'utilisation d'un tel applicateur, l'utilisateur du dispositif selon l'invention, le patient lui-même ou le personnel soignant l'animal, est plus enclin à respecter le protocole de traitement. Or, l'observance d'un traitement est un élément capital dans le succès d'un traitement.

On notera que le film pourra être fourni indépendamment de l'applicateur, l'utilisateur ayant alors à positionner le film sur celui-ci avant de mettre en œuvre le dispositif.

Il sera également possible de prévoir des dispositifs à usage unique, dans lesquels le film sera déjà placé entre le corps éjecteur et le corps principal de l'applicateur. Ce placement pourra être effectué de manière automatisée, lors de la production industrielle du dispositif.

De tels films sont particulièrement adaptés à la libération de molécules d'intérêt dans l'organisme. De préférence, ces molécules sont des principes actifs pharmaceutiques, comme les hormones, les spermicides, les contraceptifs, les antibiotiques, les antifongiques, les anesthésiques etc.... La libération de manière ciblée et locale de tels principes actifs permet d'éliminer nombre d'effets secondaires néfastes des traitements administrés par voie générale. Ainsi, la quantité de principes actifs à incorporer à la formulation de tels films est considérablement diminuée, tout en conservant la même efficacité. Le traitement devient par conséquent moins onéreux, pour le consommateur et/ou les caisses de santé publique.

Le caractère biodégradable de tels films leur confère en outre de nombreux avantages. Notamment, la dégradation du film est interne à la cavité et ne produit par conséquent aucun déchet susceptible de devoir être retirés ou éliminés.

Enfin, le dispositif selon l'invention se prête également aux applications vétérinaires, par exemple pour le traitement de pathologies vétérinaires ou l'amélioration de la fertilité chez une femelle reproductrice.

Préférentiellement ledit corps éjecteur et ledit corps principal présentent chacun une section transversale de forme ellipsoïdale. Une telle forme ellipsoïdale correspond à une forme en cylindre plus ou moins aplati du corps éjecteur et du corps principal.

Une telle forme permet, comme il apparaîtra plus précisément dans la suite de la présente description, dans un premier temps de conformer le film correctement et facilement lorsqu'on le place sur l'applicateur, et dans un second temps le dépliement correct du film, sans déchirement ni froissement, au sein de la cavité naturelle en question. Ainsi l'utilisation d'un tel dispositif est considérablement simplifiée pour l'utilisateur. L'observance du traitement proposé par la composition peut être sensiblement améliorée.

Dans un mode de réalisation avantageux, le film biodégradable se présente préférentiellement sous la forme d'une languette, c'est-à-dire de forme mince étroite et allongée. Une telle forme de languette est notamment particulièrement adaptée à une section ellipsoïdale de l'applicateur. L'utilisateur replie la languette dans le sens de sa longueur sur le corps éjecteur de l'applicateur, avant d'insérer l'ensemble dans le corps principal. Les avantages sont nombreux : le sens de positionnement du film sur l'applicateur est évident, aucun pli pouvant déchirer le film et former des débris n'est formé, le contact entre le film et les doigts de l'utilisateur est limité ce qui permet donc d'éviter les contaminations. La forme de languette du film pourra aussi faciliter l'automatisation de la production de dispositifs selon l'invention à usage unique.

De préférence, ladite languette présente une forme essentiellement rectangulaire. Cette forme est très simple à produire à l'échelle industrielle. La forme rectangulaire est en outre particulièrement adaptée à la forme ellipsoïdale du corps éjecteur. Elle facilite notamment le placement du film sur la surface du corps éjecteur ainsi que son introduction dans le corps principal, sans déchirure, ni froissement du film. Elle permet également un dépliement correct du film dans la cavité.

Dans un mode de réalisation davantage préféré, ladite languette présente une forme de rectangle pourvu dans sa partie médiane d'au moins deux échancrures latérales. Ainsi, les parties les plus larges de la languette sont placées à la surface du corps éjecteur tandis que la partie médiane présentant les échancrures vient recouvrir l'extrémité arrondie du corps éjecteur. Très simple à placer, le film ne bouge pas lorsqu'on introduit l'ensemble dans le corps principal.

Avantageusement, l'extrémité proximale dudit corps éjecteur est pourvu d'un premier épaulement permettant de stopper le coulissement dudit corps éjecteur à l'intérieur dudit corps principal. La présence d'un épaulement permet d'arrêter la course du corps éjecteur dans le corps principal et par là même, de contrôler le mouvement de ces différents éléments l'un par rapport à l'autre. Lors de l'application du film dans la cavité naturelle, l'utilisateur fait coulisser le corps éjecteur sur lequel le film est placé dans le corps principal tubulaire, jusqu'à faire sortir complètement le film du corps principal. La présence d'au moins un épaulement permet de bloquer l'avancée du corps éjecteur et le retenir dans le corps principal.

Préférentiellement, ledit corps tubulaire comprend en outre à son extrémité distale au moins deux lamelles orientées vers l'axe longitudinal dudit dispositif, lesdites au moins deux lamelles étant espacées entre elles et étant incurvées vers l'axe longitudinal dudit corps tubulaire et définissant un espace central. De manière plus préférentielle, la largeur de l'orifice défini par les lamelles est égale à celle du film entre les deux échancrures. Ainsi, le film ne se déchire pas lors de la libération du film. Il ne reste aucun débris de film sur le dispositif applicateur. Le nettoyage de l'applicateur s'en trouve facilité et l'utilisateur est certain que la dose de principe actif voulue a été délivrée. De plus, cette caractéristique permet de concevoir un dispositif dont la forme générale est ergonomique. Ainsi, l'introduction du dispositif selon l'invention dans une cavité naturelle est plus confortable et moins douloureuse. Elle permet en outre de limiter tout risque de blessure.

Dans un mode de réalisation avantageux, les dimensions dudit corps principal sont ajustées à celles dudit corps éjecteur. Ceci évite toute déformation du film entre ces deux éléments.

De préférence, ledit corps éjecteur comprend une portion distale et une portion proximale séparées par un second épaulement, ladite portion distale étant destinée à accueillir ledit film biodégradable. La présence d'un second épaulement délimitant la portion distale permet d'indiquer à l'utilisateur où et comment positionner le film sur l'applicateur. De plus, cet épaulement permet de maintenir le film en place et l'empêche de glisser le long du corps éjecteur lorsque ce dernier est introduit dans le corps principal. Enfin, il est particulièrement intéressant, lors de l'assemblage du corps éjecteur avec le film puis le corps principal, de pouvoir bloquer le corps éjecteur à l'intérieur du corps principal. Cela permet de ne pas avoir à le maintenir pendant que le dispositif est introduit dans le corps. La préhension et l'application du film s'en trouvent donc particulièrement facilitées pour l'utilisateur.

Dans une variante préférée, ladite portion proximale présente une section transversale supérieure à la section transversale de ladite portion distale.

Avantageusement, ledit corps éjecteur comprend au moins un renforcement s'étendant au moins en partie sur une longueur latérale dudit corps éjecteur. De tels renforcements permettent de guider plus facilement le corps éjecteur lorsqu'il est introduit dans le corps principal. Ils contribuent en outre au maintien du film en place, en l'empêchant de glisser sur les côtés et d'être soumis à un mouvement de torsion lors de l'introduction du corps éjecteur et du film dans le corps principal. Cela permet donc d'éviter, entre autres désagréments, le déchirement du film dans l'applicateur.

Selon un mode de réalisation, ledit corps principal comprend en outre à son extrémité proximale des moyens facilitant la préhension dudit dispositif.

De préférence, la surface externe dudit corps principal est au moins en partie pourvu de reliefs. De tels moyens permettent de faciliter la prise en main du dispositif et de simplifier l'application du film dans la cavité. Ainsi, le geste d'application est simple, rapide et sûr. L'automatisme s'installe rapidement chez l'utilisateur, le soin devient alors un geste routinier ce qui facilite et augmente l'observance du traitement.

L'applicateur du dispositif selon l'invention sera préférentiellement réalisé en un matériau plastique non allergène et résistant à la stérilisation, tel que le polyéthylène, le polypropylène, le polyéthylène téraphtalate... Ce matériau pourra notamment être avantageusement lui même biodégradable et peut être choisi parmi les polymères à base d'amidon, le polyhydroxyalkanoate (PHA), le polyhydroxybutyrate (PHB), le polyhydroxyvalerate (PHV), l'acide polylactique (PLA), un polymère à base de cellulose, le chitosan, un polypeptide, le polycaprolactone (PLC), le polyester amide (PEA), le copolyester aliphatique (PBSA) et les membres de la famille des polyesters. Cette variante sera particulièrement intéressante lorsque le dispositif sera à usage unique.

Le dispositif selon l'invention permet de déposer un film dans une cavité naturelle notamment vaginale, mais aussi rectale ou nasale. Les dimensions du dispositif selon l'invention seront déterminées en fonction de l'utilisation et de la nature de cette cavité.

Pour une variante selon laquelle le dispositif selon l'invention est destiné à être utilisé chez l'animal, le corps éjecteur présente une longueur comprise entre 30 mm et 900 mm, un plus grand diamètre compris entre 3 mm et 200 mm et un plus petit diamètre compris entre 2 mm et 190 mm. Dans cette variante, le corps principal présente une longueur comprise entre 15 mm et 600 mm, un plus grand diamètre compris entre 4 mm et 201 mm et un plus petit diamètre compris entre 3 mm et 191 mm. Selon une autre variante, lorsque le dispositif selon l'invention est destiné à une application humaine, le corps éjecteur présente une longueur comprise entre 30 mm et 200 mm, un plus grand diamètre compris entre 3 mm et 50 mm et un plus petit diamètre compris entre 2 mm et 49 mm. Dans cette variante, le corps principal présente une longueur comprise entre 15 mm et 150 mm, un plus grand diamètre compris entre 4 mm et 51 mm et un plus petit diamètre compris entre 3 mm et 50 mm.

Ces dimensions permettent notamment de prévoir différentes destinations pour le dispositif selon l'invention chez l'humain et notamment des applications nasales, vaginales, anorectales ou buccales. Ces dimensions sont de plus parfaitement adaptées pour des utilisations chez l'animal tel que les bovins, les ovins, les équidés ...

### 5. Liste des figures

D'autres caractéristiques et avantages de l'invention apparaîtront plus clairement à la lecture de la description suivante d'un mode de réalisation destiné à l'application d'un film biodégradable dans le vagin, donné à titre de simple exemple illustratif et non limitatif, et des dessins annexés, parmi lesquels :
- la figure 1 est une vue en perspective éclatée de ce mode de réalisation;
- la figure 2 est une coupe longitudinale du dispositif selon la figure 1 en position assemblée ;
- la figure 3 présente une coupe longitudinale du dispositif selon la figure 1 en position d'application ;
- la figure 4 est une coupe longitudinale du corps éjecteur du dispositif selon la figure 1 ;
- la figure 5 représente le patron du film biodégradable du dispositif selon la figure 1 ;
- la figure 6 est une coupe longitudinale du corps principal du dispositif selon la figure 1.

### 6. Description d'un mode de réalisation de l'invention

Le principe général de l'invention repose sur la conception d'un dispositif comprenant un film biodégradable incluant au moins un principe actif et un applicateur comprenant un corps éjecteur et un corps principal. Les deux corps formant l'applicateur ont préférentiellement des sections transversales de forme ellipsoïdale et le film a préférentiellement une forme de languette. Ces sections et formes permettent de disposer facilement le film sur les faces aplaties du corps éjecteur. Dans une variante intéressante, l'applicateur selon l'invention est réalisé en un matériau plastique, de préférence issu de la chimie verte et biodégradable. Ainsi, le dispositif entier est plus respectueux de l'environnement.

### 6.1. Applicateur d'un film biodégradable pour une indication gynécologique.

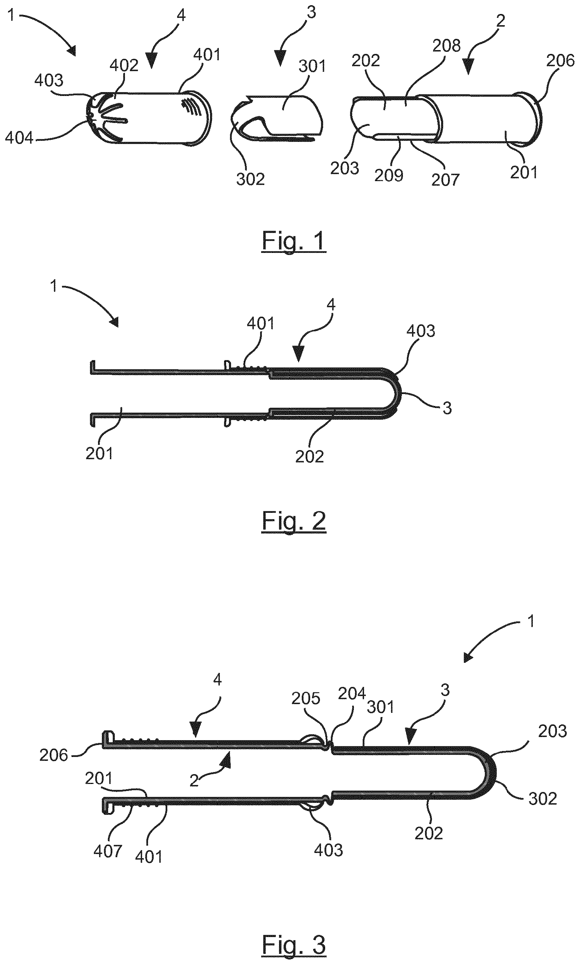

On présente, en relation avec la figure 1, une vue en éclaté et en perspective d'un mode de réalisation du dispositif selon l'invention.

Le dispositif sanitaire 1 comprend un applicateur et un film. L'applicateur est réalisé en matériau plastique choisi parmi le polyéthylène, le polypropylène et le polyéthylène téraphtalate. L'applicateur comprend un corps éjecteur 2 de section transversale de forme ellipsoïdale, un film biodégradable 3, et un corps principal 4 de section transversale de forme ellipsoïdale.

Le film biodégradable 3 vient recouvrir le corps éjecteur 2 et l'ensemble ainsi formé est ensuite introduit dans la partie proximale 401 du corps principal 4.

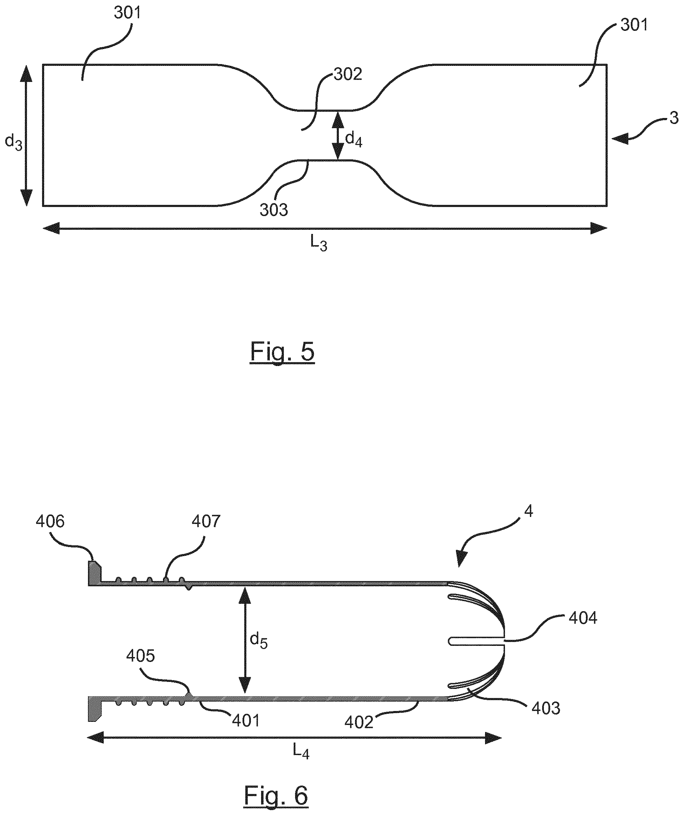

Les dimensions du film sont adaptées à celle de l'applicateur. Notamment, en référence à la figure 5, le film 3, dans ce mode de réalisation, est une languette qui présente une forme substantiellement rectangulaire et qui comprend deux échancrures 303 dans sa partie médiane 302. Le film est replié au niveau de la partie médiane 302, de moindre largeur, pour venir recouvrir le corps éjecteur 2. Plus précisément, les bords larges 301 de la languette 3 viennent recouvrir les faces supérieures 208 et inférieure 207 du corps éjecteur, tandis que la partie de moindre largeur 302 vient recouvrir l'extrémité distale 203 du corps éjecteur 2. Le corps éjecteur présente, dans ce mode de réalisation, des renforts latéraux 209 et un épaulement annulaire 204 au niveau de sa partie médiane. Les renforts latéraux 209 prennent ici la forme d'un épaulement s'étendant sur toute la longueur latérale de la portion distale 202 du corps éjecteur 2. Ces différents éléments ont notamment pour utilité :
- de guider le corps éjecteur 2 lorsqu'il coulisse dans le corps principal 4, et
- de stabiliser le film 3 lorsqu'il est situé sur le corps éjecteur afin d'éviter qu'il ne glisse, ne se déplace ou ne se torde lors de l'introduction de l'ensemble corps 2 - film 3 dans le corps principal 4.

Le corps principal 4 est pourvu, à son extrémité distale, d'un ensemble de lamelles 403 souples définissant un orifice 404. En référence aux figures 1 et 5, la largeur d4 du film entre ses échancrures est égale ou inférieure à celle de l'orifice 403. De même, la largeur d3 du film 3 au niveau des ses bords larges 301 est sensiblement égale au plus grand diamètre de la section ellipsoïdale du corps éjecteur 2.

La figure 2 est une coupe longitudinale du dispositif selon la figure 1 en position assemblée lorsque l'utilisatrice procède au montage du dispositif ou lorsqu'elle se procure le dispositif déjà assemblé. On observe que la portion distale 202 du corps éjecteur est recouverte du film 3, le tout étant introduit dans le corps principal 4. Au moment d'appliquer le film, l'utilisatrice exerce une poussée sur la partie proximale du corps 2, afin de le faire coulisser à l'intérieur du corps 4. Le dispositif est pourvu de moyens de blocage permettant d'arrêter le coulissement du corps éjecteur dans le corps principal.

Comme on le voit à la figure 3, lorsque le dispositif se trouve en position d'application, la portion proximale 201 du corps éjecteur 2 est à l'intérieur du corps principal 4. La portion distale 202, sur laquelle est positionné le film 3, se retrouve alors entièrement sortie du corps principal 4. Le film 3, qui est juste posé et qui n'adhère pas au corps éjecteur 2, s'en détache naturellement et reste dans la cavité naturelle. Les principes actifs qu'il contient peuvent alors être libérés dans l'organisme.

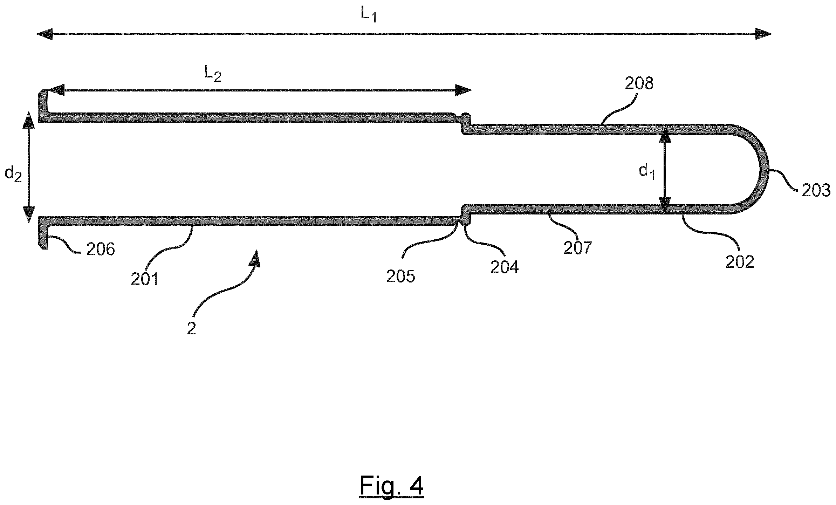

Une vue plus précise du corps éjecteur est représentée à la figure 4. Le corps éjecteur 2 est tubulaire, creux, et présente une section transversale ellipsoïdale définie par un plus grand diamètre et un plus petit diamètre. Le plus grand diamètre, qui correspond à la largeur du corps éjecteur 2, est dans ce mode de réalisation de 20 mm. Le corps 2 présente, dans sa partie médiane, un épaulement annulaire 204 qui délimite dans le corps 2 une portion proximale 201 ainsi qu'une portion distale 202. L'extrémité 203 de la portion distale 202 est arrondie pour plus d'ergonomie. Le corps éjecteur 2 présente une paroi dont l'épaisseur est d'environ 1 mm. La longueur de la portion proximale 201 est de 54 mm et son diamètre le plus petit d2 est égal à 14 mm. Le diamètre le plus petit d1 de la portion distale 202 est égal à 11 mm. L'épaulement annulaire 204 est adjacent à une gorge annulaire 205, destinée à coopérer avec le corps principal 4. La longueur totale L1 du corps éjecteur 2 est égale à 92 mm, tandis que la longueur L2 de la portion proximale est égale à 54 mm. Le corps 2 comprend en outre à son extrémité proximale un épaulement 206. Cet épaulement permet notamment de faciliter la préhension du corps par l'utilisateur, lorsqu'il manipule le dispositif selon l'invention et en particulier lorsqu'il applique le film 3 dans une cavité de son organisme.

On présente en relation avec la figure 5 un patron du film biodégradable 3. Ce film se présente sous la forme d'une languette. Ce film comprend au moins un principe actif thérapeutique, par exemple un anti-fongique, un antibiotique, un agent spermicide etc. Le film 3 présente une forme substantiellement rectangulaire comprenant deux échancrures 303 dans sa partie médiane 302, c'est-à-dire que sa forme générale s'inscrit dans un rectangle de largueur d3 et de longueur L3. La longueur totale L3 du film est d'environ 80 mm. L'épaisseur du film est approximativement d'1 mm. La largeur d3 des bords 301 est égale à 20 mm, c'est-à-dire qu'elle est sensiblement égale au plus large diamètre de la section ellipsoïdale du corps éjecteur. La largeur entre les échancrures d4 est d'environ 7 mm. Cette largeur est mesurée entre les côtés parallèles des échancrures.

La figure 6 présente une vue de côté du corps principal 4. Dans ce mode de réalisation, le corps principal présente une longueur totale L4 de 53 mm et un diamètre d5 de 14 mm. Le corps principal présente une ouverture à son extrémité proximale permettant d'introduire le corps éjecteur 2 recouvert du film 3 lors de l'assemblage du dispositif, ainsi qu'un orifice 404 à son extrémité distale 402 permettant d'appliquer le film 3. Comme indiqué précédemment, le corps principal est pourvu d'une pluralité de lamelles souples 403. Ces lamelles définissent à leur sommet l'orifice 404. Elles sont également espacées les unes des autres, ce qui permet leur mouvement relatif lors du coulissement du corps 2 dans le corps 4. Leur souplesse est notamment obtenue grâce à une épaisseur moindre par rapport à l'épaisseur des portions proximale 401 et distale 402 du corps principal 4. Cette souplesse des lamelles permet de faire sortir le corps éjecteur supportant le film facilement, sans déchirer, froisser ou retenir le film à l'intérieur du corps principal. Ainsi l'application du film biodégradable à usage gynécologique s'en trouve particulièrement simplifiée. Le corps 4 présente en outre une partie en relief 407 sur la surface externe de sa portion proximale 401. Cette partie en relief 407 permet d'éviter tout dérapage ou glissement du dispositif entre les mains de l'utilisatrice. Le geste est donc plus sûr, plus rapide, plus simple et plus précis. L'utilisatrice ne ressent donc aucune appréhension ou contrariété à suivre son traitement.

Un autre avantage du dispositif selon l'invention est la présence de moyens de blocage du mouvement relatif du corps éjecteur 2 dans le corps principal 4. Ces moyens comprennent notamment la présence d'épaulements aux extrémités proximales du corps éjecteur 2 et du corps principal 4. Dans ce mode de réalisation, l'épaulement 406 du corps principal bute contre l'épaulement 206 du corps éjecteur lorsque le dispositif est dans sa position d'application. La course du corps éjecteur à l'intérieur du corps principal est alors contrôlée par des moyens mécaniques. De plus, le corps principal 4 peut présenter un épaulement 405 dans sa partie proximale 401. Cet épaulement 405 est destiné à coopérer avec la gorge annulaire 205 du corps éjecteur 2 lorsque le dispositif selon l'invention est assemblé, avant application du film. Ainsi le corps principal 4 est maintenu autour du corps 2 et du film 3, avant toute utilisation du dispositif selon l'invention. Cela permet de protéger le film avant son application.

Ce blocage de nature mécanique n'est toutefois pas irréversible. Une légère pression exercée par l'utilisatrice sur l'épaulement 206 du corps éjecteur 2 permet de dégager l'épaulement 405 de la gorge annulaire 205, et de faire coulisser le corps éjecteur 2 dans le corps principal 4.

### 6.2. Variantes

Le corps éjecteur selon l'invention peut présenter la forme d'un piston. La partie distale est alors remplacée par une tige, terminée par un poussoir suffisamment large pour buter contre l'épaulement proximal 406 du corps principal.

Le dispositif selon l'invention peut également être utilisé pour des applications rectales, aussi bien chez l'enfant que chez l'adulte. Dans ce cas, le film peut contenir différentes substances dont il est nécessaire qu'elles soient rapidement absorbées par l'organisme, par exemple les anti-nauséeux.

Des applications nasales sont également intéressantes pour décongestionner les sinus. Il est en effet fréquent que les gouttes et les sprays aient des efficacités limitées du fait que le liquide coule et ne reste pas suffisamment en contact avec la paroi nasale. De plus, l'application d'un film permet de solutionner les problèmes de tonicités souvent trop importantes des solutions nasales.

Un dispositif applicateur selon l'invention peut également trouver son utilité pour traiter les épitaxies bénignes ou graves. L'administration d'un spray nasal peut parfois suffire pour arrêter un léger saignement du nez. Toutefois, le traitement de choix en cas de saignement important est le tamponnement. Il existe deux méthodes de tamponnement :
- le tamponnement antérieur : la mèche est introduite dans la narine ; et
- le tamponnement postérieur : la mèche est introduite dans l'arrière gorge et est ensuite retirée. Cette ablation de la mèche peut parfois causer des irritations, à l'origine d'une récidive de l'épitaxie.
L'utilisation de films biodégradables et contenant des substances vasoconstrictrices permettent donc de faire cesser les saignements du nez, quelle que soit leur importance. De plus, de part leur biodégradabilité, les films se délitent spontanément et permettent donc d'éviter tous les inconvénients liés à l'ablation d'une mèche. A titre d'exemple de substances vasoconstrictrices pouvant être contenues dans un tel film, on peut citer les bicarbonates, l'angiotensine II, l'adrénaline, la noradrénaline, l'hormone antidiurétique, l'endothéline ...Les films biodégradables peuvent également contenir des substances cicatrisantes pour guérir la plaie plus rapidement et éviter les récidives.

Un autre exemple d'utilisation du dispositif selon l'invention est celui d'un applicateur buccal pour soins ondoto-stomatologiques. Les films déposés à l'aide de tels applicateurs peuvent contenir :
- des anesthésiques pour une anesthésie locale précédent l'acte médical; ou
- des substances vasoconstrictrices pour faire cesser les saignements.
Ainsi la douleur de l'injection d'un anesthésique local dans une gencive enflammée est évitée. Le risque d'infection lié à la pénétration de l'aiguille dans la gencive est également supprimé. De plus, lorsque la gencive saigne pendant le soin, le praticien choisit soit d'aspirer le sang, soit de poser une mèche pour absorber et tamponner la plaie. L'application d'un film vasoconstricteur et/ou cicatrisant à la surface de la plaie gingivale permet de faire cesser le saignement rapidement.

Le dispositif selon l'invention convient également pour l'application d'un film à destination ano-rectale. Un tel dispositif peut donc servir pour prévenir ou traiter des pathologies telles que les hémorroïdes, les fissures anales, les abcès, les suintements, les infections, les maladies sexuellement transmissibles etc... Lorsque le film contient des substances chimiothérapeutiques, le dispositif selon l'invention peut également servir à traiter de manière ciblée et locale les tumeurs ano-rectales. Ainsi, le traitement de la tumeur gagne en efficacité tout en limitant les impacts négatifs de la chimiothérapie sur l'organisme entier du patient.

Le dispositif selon l'invention est également adapté aux traitements et aux soins vétérinaires, en particulier pour l'application intra-vaginale d'hormones sexuelles pour améliorer ou traiter un problème de fertilité.

Les dimensions du dispositif selon l'invention peuvent être adaptées en fonction de la destination anatomique, de la taille et de l'âge du patient en utilisant les connaissances générales de l'homme du métier.

## Revendications

1. Dispositif sanitaire (1) d'application d'au moins un principe actif à l'intérieur d'une cavité naturelle humaine ou animale,ledit dispositif comprenant au moins un film biodégradable (3) contenant ledit au moins un principe actif et un applicateur incluant :
- un corps principal tubulaire (4) présentant une première ouverture à son extrémité proximale (401) et une seconde ouverture à son extrémité distale (402); et
- un corps éjecteur (2) comprenant une portion distale (202) dont au moins une partie de la surface forme un support pour l'au moins un film biodégradable (3) et dont l'extrémité (203) est arrondie,
ledit corps éjecteur (2) étant mobile de manière coulissante à l'intérieur dudit corps principal (4) entre au moins une position dans laquelle ladite portion distale (202) dudit corps éjecteur (2) se trouve à l'intérieur dudit corps principal (4) et une position d'application dans laquelle ladite portion distale (202) se trouve à l'extérieur dans le prolongement axial dudit corps principal (4).

2. Dispositif selon la revendication 1 **caractérisé en ce que** ledit corps éjecteur (2) et ledit corps principal (4) présentent chacun une section transversale de forme ellipsoïdale.

3. Dispositif selon la revendication 1 ou 2 caractérisé en ce ledit film biodégradable (3) se présente sous la forme d'une languette.

4. Dispositif selon la revendication 3 **caractérisé en ce que** ladite languette présente une forme essentiellement rectangulaire.

5. Dispositif selon la revendication 4 **caractérisé en ce que** ladite languette présente une forme de rectangle pourvu dans sa partie médiane (302) d'au moins deux échancrures latérales (303).

6. Dispositif selon l'une des revendications précédentes dans lequel l'extrémité proximale (201) dudit corps éjecteur (2) est pourvue d'un premier épaulement (206) permettant de stopper le coulissement dudit corps éjecteur (2) à l'intérieur dudit corps principal (4).

7. Dispositif selon l'une des revendications précédentes dans lequel ledit corps tubulaire (4) comprend en outre à son extrémité distale (402) au moins deux lamelles (403) orientées vers l'axe longitudinal dudit dispositif, lesdites au moins deux lamelles, étant espacées entre elles et étant incurvées vers l'axe longitudinal dudit corps tubulaire, définissent un espace central (404).

8. Dispositif selon l'une quelconque des revendications précédentes dans lequel les dimensions dudit corps principal (4) sont ajustées à celles dudit corps éjecteur (2).

9. Dispositif selon l'une quelconque des revendications précédentes dans lequel ledit corps éjecteur (2) comprend une portion distale (202) et une portion proximale (201) séparées par un second épaulement (204), ladite portion distale (202) étant destinée à accueillir ledit film biodégradable (3).

10. Dispositif selon la revendication 9 **caractérisé en ce que** ladite portion proximale (201) présente une section transversale supérieure à la section transversale de ladite portion distale (202).

11. Dispositif selon l'une quelconque des revendications précédentes dans lequel ledit corps éjecteur (2) comprend au moins un renforcement (206) s'étendant au moins en partie sur une longueur latérale dudit corps éjecteur (2).

12. Dispositif selon l'une quelconque des revendications précédentes dans lequel ledit corps principal (4) comprend en outre à son extrémité proximale (401) des moyens facilitant la préhension dudit dispositif.

13. Dispositif selon l'une quelconque des revendications précédentes selon lequel la surface externe dudit corps principal est au moins en partie pourvu de reliefs (407).

14. Dispositif selon l'une quelconque des revendications précédentes **caractérisé en ce que** ledit applicateur est constitué en un matériau plastique non allergène et résistant à la stérilisation choisis parmi le polyéthylène, le polypropylène, le polyéthylène téraphtalate, un polymère à base d'amidon, le chitosan, le polyhydroxyalkanoate (PHA), le polyhydroxybutyrate (PHB), le polyhydroxyvalérate (PHV), l'acide polylactique (PLA), un polymère à base de cellulose, un polypeptide, le polycaprolactone (PLC), le polyester amide (PEA), le copolyester aliphatique (PBSA) et les membres de la famille des polyesters.

## Patentansprüche

1. Sanitärvorrichtung (1) zum Anwenden mindestens eines Wirkstoffs im Inneren einer natürlichen Körperhöhle eines Menschen oder Tieres, wobei die Vorrichtung mindestens einen bioabbaubaren Film (3), der mindestens einen Wirkstoff enthält, und einen Applikator umfasst, umfassend:
- einen rohrförmigen Hauptkörper (4), der eine erste Öffnung an seinem proximalen Ende (401) und eine zweite Öffnung an seinem distalen Ende (402) aufweist; und
- einen Ausstoßkörper (2), der einen distalen Abschnitt (202) umfasst, von dem mindestens ein Teil der Oberfläche einen Träger für den mindestens einen bioabbaubaren Film (3) bildet, und dessen Ende (203) abgerundet ist,
wobei der Ausstoßkörper (2) auf gleitende Weise im Inneren des Hauptkörpers (4) bewegbar ist zwischen mindestens einer Position, in der sich der distale Abschnitt (202) des Ausstoßkörpers (2) im Inneren des Hauptkörpers (4) befindet, und einer Anwendungsposition, in der sich der distale Teil (202) außerhalb in der axialen Verlängerung des Hauptkörpers (4) befindet.

2. Vorrichtung nach Anspruch 1, **dadurch gekennzeichnet, dass** der Ausstoßkörper (2) und der Hauptkörper (4) jeweils eine Quersektion mit ellipsoidischer Form aufweisen.

3. Vorrichtung nach Anspruch 1 oder 2, **dadurch gekennzeichnet, dass** der bioabbaubare Film (3) die Form einer Lasche aufweist.

4. Vorrichtung nach Anspruch 3, **dadurch gekennzeichnet, dass** die Lasche eine im Wesentlichen rechteckige Form aufweist.

5. Vorrichtung nach Anspruch 4, **dadurch gekennzeichnet, dass** die Lasche eine rechteckige Form aufweist, die in ihrem medianen Teil (302) mit mindestens zwei seitlichen Einbuchtungen (303) versehen ist.

6. Vorrichtung nach einem der vorhergehenden Ansprüche, wobei das proximale Ende (201) des Ausstoßkörpers (2) mit einer ersten Schulter (206) versehen ist, die es ermöglicht, das Gleiten des Ausstoßkörpers (2) im Inneren des Hauptkörpers (4) zu stoppen.

7. Vorrichtung nach einem der vorhergehenden Ansprüche, wobei der rohrförmige Körper (4) außerdem an seinem distalen Ende (402) mindestens zwei Lamellen (403) umfasst, die zur Längsachse der Vorrichtung orientiert sind, wobei die mindestens zwei Lamellen voneinander beabstandet sind und zur Längsachse des rohrförmigen Körpers gebogen sind, wodurch sie einen zentralen Raum (404) definieren.

8. Vorrichtung nach einem der vorhergehenden Ansprüche, wobei die Abmessungen des Hauptkörpers (4) an jene des Ausstoßkörpers (2) angepasst sind.

9. Vorrichtung nach einem der vorhergehenden Ansprüche, wobei der Ausstoßkörper (2) einen distalen Abschnitt (202) und einen proximalen Abschnitt (201) umfasst, die durch eine zweite Schulter (204) getrennt sind, wobei der distale Abschnitt (202) dazu bestimmt ist, den bioabbaubaren Film (3) aufzunehmen.

10. Vorrichtung nach Anspruch 9, **dadurch gekennzeichnet, dass** der proximale Abschnitt (201) eine größere Quersektion als die Quersektion des distalen Abschnitts (202) aufweist.

11. Vorrichtung nach einem der vorhergehenden Ansprüche, wobei der Ausstoßkörper (2) mindestens eine Verstärkung (206) umfasst, die sich mindestens teilweise über eine seitliche Länge des Ausstoßkörpers (2) erstreckt.

12. Vorrichtung nach einem der vorhergehenden Ansprüche, wobei der Hauptkörper (4) außerdem an seinem proximalen Ende (401) Mittel umfasst, die das Ergreifen der Vorrichtung erleichtern.

13. Vorrichtung nach einem der vorhergehenden Ansprüche, wobei die Außenfläche des Hauptkörpers mindestens teilweise mit Erhebungen (407) versehen ist.

14. Vorrichtung nach einem der vorhergehenden Ansprüche, **dadurch gekennzeichnet, dass** der Applikator aus einem nicht allergenen und sterilisationsbeständigen Kunststoffmaterial besteht, das ausgewählt ist aus Polyethylen, Polypropylen, Polyethylenterephthalat, einem Polymer auf der Basis von Stärke, Chitosan, Polyhydroxyalkanoat (PHA), Polyhydroxybutyrat (PHB), Polyhydroxyvalerat (PHV), Polymilchsäure (PLA), einem Polymer auf der Basis von Cellulose, einem Polypeptid, Polycaprolacton (PLC), Polyesteramid (PEA), aliphatischem Copolyester (PBSA) und den Mitgliedern der Familie der Polyester.

## Claims

1. Sanitary device (1) for applying at least one active ingredient inside a human or animal natural cavity, said device comprising at least one biodegradable film (3) containing at least one active ingredient and an applicator including:
- a main tubular member (4) having a first aperture at its proximal end (401) and a second aperture at its distal end (402); and
- an ejector member (2) comprising a distal portion (202), at least one part of the surface of which forms a support for the at least one biodegradable film (3), and the end (203) of which is rounded,
said ejector member (2) being slidingly mobile within said main member (4) between at least one position in which said distal portion (202) of said ejector member (2) is situated inside said main member (4) and an application position in which said distal portion (202) is situated outside in the axial extension of said main member (4).

2. Device according to claim 1 **characterized in that** said ejector member (2) and said main member (4) each have an ellipsoid-shaped cross-section.

3. Device according to claim 1 or 2 **characterized in that** said biodegradable film (3) takes the form of a tongue.

4. Device according to claim 3 **characterized in that** said tongue has an essentially rectangular shape.

5. Device according to claim 4 **characterized in that** said tongue has a rectangular shape provided in its median part (202) with at least two lateral notches (303).

6. Device according to any one of the preceding claims wherein the proximal end (201) of said ejector member (2) is provided with a first shoulder (206) allowing to stop the sliding of said ejector member (2) inside said main member (4).

7. Device according to any one of the preceding claims wherein said tubular member (4) furthermore comprises, on its distal end (402), at least two strips (403) oriented towards the longitudinal axis of said device, said at least two strips being spaced out from one another and being curved inwardly towards the longitudinal axis of said tubular member, defining a central space (404).

8. Device according to any one of the preceding claims wherein the dimensions of said main member (4) are adjusted to those of said ejector member (2).

9. Device according to any one of the preceding claims wherein said ejector member (2) comprises a distal portion (202) and a proximal portion (201) separated by a second shoulder (204), said distal portion (202) being intended to receive said biodegradable film (3).

10. Device according to claim 9, **characterized in that** said proximal portion (201) has a cross-section greater than the cross-section of said distal portion (202).

11. Device according to any one of the preceding claims wherein said ejector member (2) comprises at least one reinforcement piece (206) extending at least partly along a lateral length of said ejector member (2).

12. Device according to any one of the preceding claims wherein said main member (4) furthermore comprises, at its proximal end (401), means for facilitating the grasping of said device.

13. Device according to any one of the preceding claims according to which the external surface of said main member is at least partly provided with relief features (407).

14. Device according to any one of the preceding claims **characterized in that** said applicator is constituted by a non-allergenic and resistant to sterilization plastic material such as polyethylene, polypropylene, polyethylene teraphtalate, a arch-based polymer, chitosan, polyhydroxyalkanoate (PHA), polyhydroxybutyrate (PHB), polyhydroxyvalerate (PHV), polylactic acid (PLA), a cellulose-based polymer, a polypeptide, polycaprolactone (PLC), polyester amide (PEA), aliphatic copolyester (PBSA) and the members of the polyester family.
